# EUROPÄISCHE PATENTSCHRIFT

(11) **EP 1 089 778 B1**
(45) Veröffentlichungstag und Bekanntmachung des Hinweises auf die Patenterteilung: **29.03.2006**
(21) Anmeldenummer: 00938501.4
(22) Anmeldetag: 20.04.2000
(51) Int. Cl.: A61M 1/02

(54) **VORRICHTUNG ZUM AUFTRENNEN VON BLUT IN EINZELNE UND/ODER GRUPPEN SEINER BESTANDTEILE**
DEVICE FOR SEPARATING BLOOD INTO INDIVIDUAL COMPONENTS AND/OR GROUPS OF SAID COMPONENTS
DISPOSITIF POUR DIVISER LE SANG EN SES COMPOSANTS INDIVIDUELS ET/OU EN GROUPES DE CEUX-CI

(30) Priorität: 20.04.1999 DE 29906812 U
(43) Veröffentlichungstag der Anmeldung: 11.04.2001
(73) Patentinhaber: Heim Medizintechnik GmbH, 45966 Gladbeck (DE)
(72) Erfinder: HEIM, Gerd, D-45966 Gladbeck (DE)
(74) Vertreter: Kreuzkamp, Markus
(86) Internationale Anmeldenummer: PCT/DE2000/001258
(87) Internationale Veröffentlichungsnummer: WO 2000/062840

(56) Entgegenhaltungen:
- WO-A-95/02443
- DE-A- 3 043 682
- DE-A- 19 733 407

## Beschreibung

Die Erfindung betrifft eine Vorrichtung zum Auftrennen von Blut in einzelne und/oder Gruppen seiner Bestandteile gemäß den Merkmalen des Oberbegriffs des Schutzanspruchs 1.

Eine solche Vorrichtung ist in der DE-A-197 33 407 sowie in der druckschriftlichen Veröffentlichung "Hämatologie, Bd. 1, Aktueller Stand der Eigenbluttransfusion, Sympomed-Verlag, München 1992, S. 108 bis 113" beschrieben, die dazu dienen soll, Eigenblut von zu operierenden Spendern vor der Operation abzunehmen und in seine Bestandteile zu zerlegen, um das erhaltene Erythrozytenkonzentrat zunächst aufbewahren und während und/oder nach der Operation dem Spender zuführen zu können, ohne daß das Blut zwischenzeitlich in einer Zentrifuge behandelt werden muß und vor, während und/oder nach dieser Behandlung die Gefahr besteht, daß das Blut oder einer seiner Bestandteile mit Luft und damit auch mit Keimen in Berührung kommt. Innerhalb der vorstehend angegebenen Beschreibung wird u.a. auch erwähnt, daß es auf die richtige Anordnung der in Form von Beuteln vorliegenden Behälter ankommt, um so über den transmembranösen Filtrationsdruck die erzielte Plasmamenge zu bestimmen. Es fehlt allerdings jeder Hinweis darauf, daß - wie zwischenzeitliche Praxistests ergeben haben - bei einer Vorrichtung der in Rede stehenden Art die gegenseitige Anordnung der Behälter keineswegs nur eine Variation der erzeugten Plasmamenge bedingt, sondern allenfalls in eng begrenzten Anordnungsbereichen überhaupt eine akzeptable Auftrennung von Blut in seine Bestandteile gestattet. In keinem Falle offenbart diese Beschreibung eine Möglichkeit, die vorgenannte Vorrichtung auch im täglichen Routinebetrieb, beispielsweise bei Blutspendeaktionen des Deutschen Roten Kreuzes zur Gewinnung von Fremdblutkonserven, ohne erheblichen Aufwand zum Einrichten der Vorrichtung zu benutzen, um auch in den vorgenannten Fällen auf die risikobehaftete und arbeitsintensive Nachbehandlung der Blutkonserven mittels Zentrifuge verzichten zu können.

Darüber hinaus erwähnt die obengenannte Beschreibung zwar auch die Möglichkeit, Leukozyten abtrennen zu können, führt dies aber ebenfalls nicht durch, obwohl sich eine solche Abtrennung zwischenzeitlich als durchaus wünschenswert ergeben hat.

Aus den vorgenannten Gründen liegt der vorliegenden Erfindung die Aufgabe zugrunde, eine Vorrichtung zur Verfügung zu stellen, mit der in einem sterilisierten geschlossenen System eine möglichst weitgehende Auftrennung von Blut in einzelne und/oder Gruppen seiner Bestandteile durchführbar ist, wobei die erforderlichen Druckverhältnisse innerhalb der Vorrichtung auf einfache und reproduzierbare Weise einstellbar sind.

Die Erfindung löst diese Aufgabe mit Hilfe der Gesamtheit der Merkmale des Patentanspruchs 1.

Dabei erweist es sich als besonders vorteilhaft, daß die Filteranordnung ein mit einer blutverträglichen Flüssigkeit durchtränkter Hohlfaserfilter bekannter Art ist, wobei die Gesamtheit der Innenbereiche der Hohlfasern die Einlaßkammer und die Gesamtheit der Außenbereiche der Hohlfasern die Auslaßkammer ausbildet, daß im Leitungsweg zwischen Sammelbehälter und Aufnahmebehälter für zelluläre Bestandteile des Blutes ein Leukozytendepletionsfilter ebenfalls bekannter Art angeordnet ist und daß der Leitungsweg zwischen Filteranordnung und Aufnahmebehälter für zelluläre Bestandteile des Blutes eine Einrichtung zur Erzeugung eines Rückstaus in der Filteranordnung aufweist, weil eine solche Vorrichtung gestattet, einerseits eine eindeutige Trennung von Blutplasma und zellulären Bestandteilen des Blutes sicherzustellen, wobei der durchtränkte Hohlfaserfilter zunächst die gewünschte Filterfunktion garantiert und die Einrichtung zur Erzeugung eines Rückstaus in der Filteranordnung entweder eine einmalige Festeinstellung für eine ganze Serie von Blutaufnahmen oder von Fall zu Fall zumindest ein einfaches Nachjustieren von Hand ermöglicht, und andererseits aus der Gesamtheit der zellulären Bestandteile des Blutes in jedem Fall auch noch die Leukozyten gesondert zu separieren.

Vorteilhafte Ausgestaltungen des erfindungsgemäßen Gegenstandes des Patentanspruchs 1 werden durch den Inhalt der Unteransprüche 2 bis 34 offenbart.

Ein Ausführungsbeispiel der erfindungsgemäßen Vorrichtung ist in der Zeichnung dargestellt.

Es zeigen:
- Fig. 1:: Ansicht einer Vorrichtung zum Auftrennen von Blut in einzelne und/oder Gruppen seiner Bestandteile in schematischer Darstellung.
- Fig. 2:: Ansicht einer anderen Ausführungsform einer Vorrichtung zum Auftrennen von Blut in einzelne und/oder Gruppen seiner Bestandteile in schematischer Darstellung.
- Fig. 3:: Ansicht einer weiteren Ausführungsform einer Vorrichtung zum Auftrennen von Blut in einzelne und/oder Gruppen seiner Bestandteile in schematischer Darstellung.
- Fig. 4:: Ansicht einer vierten Ausführungsform einer Vorrichtung zum Auftrennen von Blut in einzelne und/oder Gruppen seiner Bestandteile in schematischer Darstellung.
- Fig. 5:: Aufsicht auf den Querschnitt durch einen Hohlfaserfilter in schematischer Darstellung.
- Fig. 6:: Schematische isometrische Darstellung einer unveränderlichen Haltevorrichtung für vorgegebene Positionen einer Vorrichtung zum Auftrennen von Blut in einzelne und/oder Gruppen seiner Bestandteile

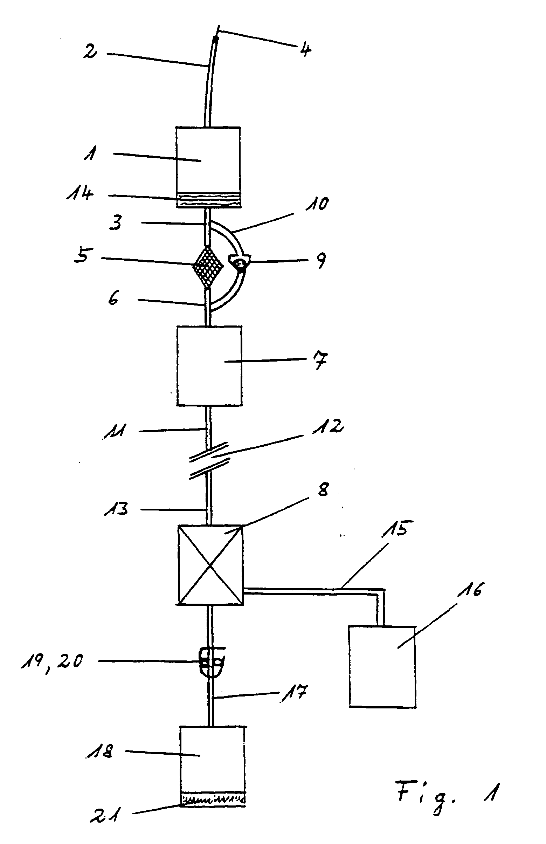

Die Fig. 1 zeigt die Ansicht einer Vorrichtung zum Auftrennen von Blut in einzelne und/oder Gruppen seiner Bestandteile in schematischer Darstellung, die zunächst aus einem hier - aber nicht zwangsläufig - als beutelartiger Hohlkörper aus Kunststoff-Folie bekannter Art vorausgesetzten Sammelbehälter 1 besteht, der eine luft- und keimdicht mit ihm verbundene Zuleitung 2 und eine ebensolche Ableitung 3 aufweist, von denen - wie auch für alle weiteren noch zu beschreibenden Verbindungsleitungen - ohne Beschränkung der Allgemeinheit vorausgesetzt ist, daß sie schlauchartige Verbindungselemente aus elastischem Kunststoffmaterial bekannter Art sind. Die Zuleitung 2 trägt an ihrem dem Sammelbehälter 1 abgewandten Ende ein mit einem oder mehreren Blutgefäßen eines Spenders koppelbares Verbindungselement 4, von dem hier - aber nicht zwingend - angenommen ist, daß es sich dabei um eine Kanüle herkömmlicher Art handelt. Die Ableitung 3 führt zu einem Leukozytendepletionsfilter 5 bekannter Art, mit dem sie ebenfalls luft- und keimdicht verbunden ist, was gleichermaßen für ihre Verlängerung 6 gilt, die im vorliegenden - aber nicht zwangsläufigen - Fall zu einem Transferbehälter 7 führt, mit dem sie ebenfalls luft- und keimfrei verbunden ist und von dem ohne Beschränkung der Allgemeinheit vorausgesetzt wird, das sein materieller Aufbau demjenigen des Sammelbehälters 1 entspricht, was im weiteren auch bezüglich aller noch folgenden Behälter gelten soll.

Die Anordnung des Leukozytendepletionsfilters 5 an der gezeigten Stelle ist nicht zwingend vorgegeben, weist jedoch den Vorteil auf, daß die nachgeschaltete und im folgenden noch im Detail diskutierte Filteranordnung 8 weniger belastet wird und daher eine schnellere Auftrennung zwischen Blutplasma und verbliebenen zellularen Bestandteilen des Blutes ermöglicht. Parallel zum Leukozytendepletionsfilter 5 ist hier zwischen Ableitung 3 und deren Verlängerung 6 - allerdings nicht zwangsläufig - eine mit einem Rückschlagventil 9 versehene Bypass-Leitung 10 angeordnet, wobei das Rückschlagventil 9 so angeordnet ist, daß es in der der normalen Fließrichtung des Blutes durch den Leukozytendepletionsfilter 5 entgegengesetzten Richtung öffnet. Diese Anordnung gestattet es beispielsweise, den Transferbehälter 7 ohne Aufwirbeln der im Leukozytendepletionsfilter 5 abgelagerten Leukozyten zu entlüften oder auch nach dem Abtrennen des Sammelbehälters 1 auf einfache Weise eine Probe zu entnehmen. Der Transferbehälter 7 muß ebenfalls nicht zwingend in der in Rede stehenden Vorrichtung angeordnet sein, bringt jedoch in bestimmten Fällen den Vorteil mit sich, daß die Blutentnahme und die Auftrennung des Blutes in einzelne und/oder Gruppen seiner Bestandteile mittels einer zeitweisen Zwischenlagerung den jeweiligen Arbeitsabläufen flexibel angepaßt werden kann.

Der Transferbehälter 7 ist über eine Verbindungsleitung 11 luft- und keimdicht mit einem Teil einer sterilen Kupplungsvorrichtung 12 bekannter Art, beispielsweise einer SCD-Anordnung, verbunden, der ein entsprechendes Gegenstück gegenübersteht, das über eine Verbindungsleitung 13 luft- und keimdicht mit einer Filteranordnung 8 verbunden ist. Die Filteranordnung 8 besteht - was in der schematischen Darstellung nicht explizit gezeigt ist - aus einem mit einer blutverträglichen Flüssigkeit, beispielsweise physiologischer Kochsalzlösung oder einer Stabilisatcrlösung 14 bekannter Art, wie sie üblicherweise bereits vor der Sterilisation der vorliegenden Vorrichtung in vorgegebener Menge in den Sammelbehälter 1 eingebracht wird, durchtränkten Hohlfaserfilter, beispielsweise einem solchen aus dem Material Akzo-Nobel Micro PES TF7, wobei die Gesamtheit der Innenbereiche der Hohlfasern eine Einlaßkammer und die Gesamtheit der Außenbereiche der Hohlfasern eine Auslaßkammer ausbilden. Die Auslaßkammer ist dann über eine luft- und keimdicht mit ihr verbundene Ablaufleitung 15 mit einem Aufnahmebehälter für Blutplasma 16 verbunden, der ebenfalls luft- und keimdicht an die Ablaufleitung 15 anschließt ; die Einlaßkammer ist ihrerseits über eine Ablaufleitung 17 mit einem Aufnahmebehälter für zelluläre Bestandteile des Blutes 18 verbunden, wobei selbstverständlich auch hier alle Verbindungsstellen luft- und keimdicht ausgeführt sind. Die sterile Kupplungsvorrichtung 12 kann selbstverständlich sowohl bereits während der Blutaufnahme verriegelt sein als auch bei Bedarf erst zu einem späteren Zeitpunkt verriegelt werden.

Die Ablaufleitung 17 ist außerdem mit einer einstellbaren Klemmvorrichtung 19 versehen, von der hier - aber nicht zwangsläufig - angenommen ist, daß es sich um eine Rollklemme bekannter Art handelt. Diese Rollklemme dient als Einrichtung zur Erzeugung eines Rückstaus 20 in der Filteranordnung 8, mit der auf einfache Weise deren einwandfreie Funktion gesteuert wird. Eine solche Einrichtung 20 kann selbstverständlich auch durch jede andere gleichwirkende Maßnahme verwirklicht werden, beispielsweise durch eine Anordnung des Leukozytendepletionsfilters 5 in der Ablaufleitung 17, gegebenenfalls in Verbindung mit einer Anordnung aller Behälter 1, 7, 16, 18 und Filtereinrichtungen 5, 6 in vorgegebenen gegenseitigen senkrechten Abständen.

Der Aufnahmebehälter für zelluläre Bestandteile des Blutes 18 wird im übrigen üblicherweise bereits vor der Sterilisation der vorliegenden Vorrichtung mit einer vorgegebenen Menge einer Additivlösung 21 bekannter Art vorgefüllt, die bei Verwendung eines Transferbehälters 7 allerdings auch bereits in diesen eingefüllt sein kann, und zwar sowohl zusätzlich als auch statt dessen.

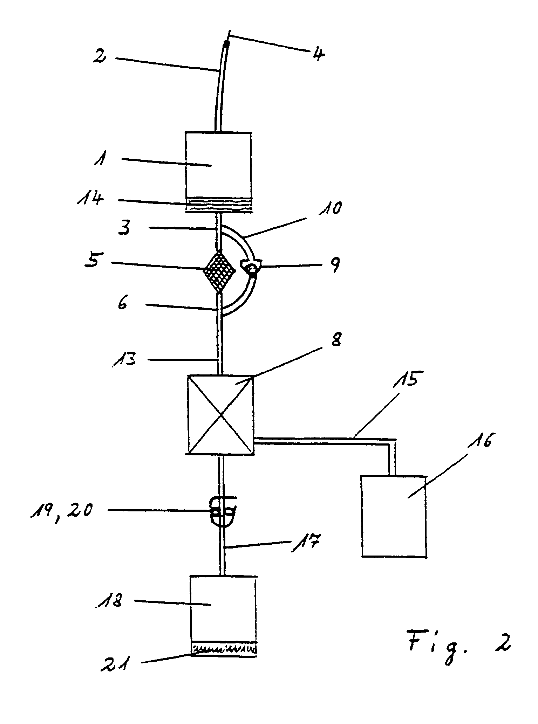

Die Fig. 2 zeigt eine Ausführungsform einer Vorrichtung zum Auftrennen von Blut in einzelne und/oder Gruppen seiner Bestandteile in schematischer Darstellung, bei der im Gegensatz zum Gegenstand der Fig. 1 sowohl auf den Transferbehälter 7 als auch auf die sterile Kupplungsvorrichtung 12 verzichtet worden ist. Dies führt zwar zu einer Einschränkung der Anpassung an die jeweiligen Arbeitsabläufe, vereinfacht aber andererseits den Gesamtaufbau und die Handhabbarkeit der vorliegenden Anordnung.

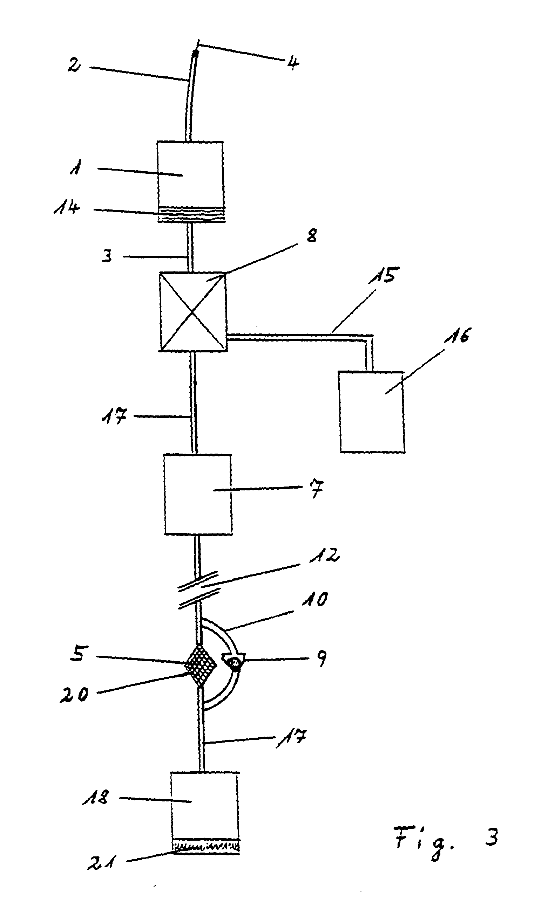

Die Fig. 3 stellt in schematischer Darstellung eine Ausführungsform einer Vorrichtung zum Auftrennen von Blut in einzelne und/oder Gruppen seiner Bestandteile dar, bei der gegenüber den Gegenständen der Fig. 1 und 2 der Leukozytendepletionsfilter 5 einschließlich der Bypass-Leitung 10 mit dem Rückschlagventil 9 aus der Stellung zwischen Sammelbehälter 1 und Filteranordnung 8 in eine Stellung zwischen Filteranordnung 8 und Aufnahmebehälter für zelluläre Bestandteile des Blutes 18 in die Ablaufleitung der Einlaßkammer 17 umgeordnet ist, wobei er gleichzeitig die einstellbare Klemmvorrichtung 19 als Einrichtung zur Erzeugung eines Rückstaus 20 ersetzt - jedenfalls dann, wenn gleichzeitig fest vorgegebene senkrechte Abstände zwischen Filteranordnung 8, Aufnahmebehälter für Blutplasma 16, Aufnahmebehälter für zelluläre Bestandteile des Blutes 18 und Leukozytendepletionsfilter 5 eingehalten werden, beispielsweise mittels einer - hier nicht im Detail dargestellten, da dem Fachmann bekannten - unveränderlichen Haltevorrichtung in Form eines starren Gestells oder Gerüstes. Außerdem ist der Transferbehälter 7 nicht mehr vor, sondern hinter der Filteranordnung 8 in der Ablaufleitung der Einlaßkammer 17 für die zellulären Bestandteile des Blutes angeordnet und die sterile Kupplungsvorrichtung 12 zwischen Transferbehälter 7 und Leukozytendepletionsfilter 5 ebenfalls in der Ablaufleitung der Einlaßkammer 17. Damit wird zwar auf die Möglichkeit verzichtet, die Filteranordnung 8 zu entlasten und einen größeren Durchsatz von leukozytenfreiem Blut zu erzielen, dafür jedoch, wenn dies beispielsweise aus räumlichen Gründen vorteilhaft erscheint, die Installation einer speziellen Einrichtung zur Erzeugung eines Rückstaus 20 eingespart, da deren Funktion gemäß vorstehender Ausführungen von dem Leukozytendepletionsfilter 5 selbst wahrgenommen werden kann, und außerdem der Vorteil gegeben ist, daß die Abtrennung der Leukozyten aus den zellulären Bestandteilen des Blutes zeitlich unabhängig von der Abtrennung des Blutplasmas aus dem ursprünglich gewonnenen Blut erfolgen kann.

Selbstverständlich schließt dies nicht aus, vor oder hinter dem Leukozytendepletionsfilter 5 eine zusätzliche einstellbare Klemmvorrichtung 19, beispielsweise in Form einer Rollklemme bekannter Art, als weitere Einrichtung zur Erzeugung eines Rückstaus 20 anzunordnen.

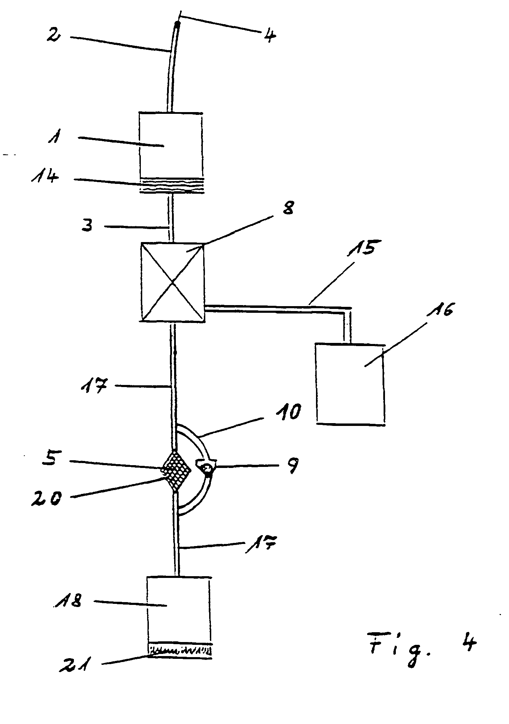

Die Fig. 4 zeigt dagegen die Ansicht einer Vorrichtung zum Auftrennen von Blut in einzelne und/oder Gruppen seiner Bestandteile in schematischer Darstellung, bei der gegenüber dem Gegenstand der Fig. 3 sowohl auf den Transferbehälter 7 als auch auf die sterile Kupplungsvorrichtung 12 verzichtet worden ist. Dies schränkt zwar ebenfalls - wie bereits beim Gegenstand der Fig. 2 - die flexible Anpassung der Vorrichtung an die erforderlichen Arbeitsabläufe ein, vereinfacht dafür aber ebenso den Gesamtaufbau und die Handhabbarkeit der vorliegenden Anordnung.

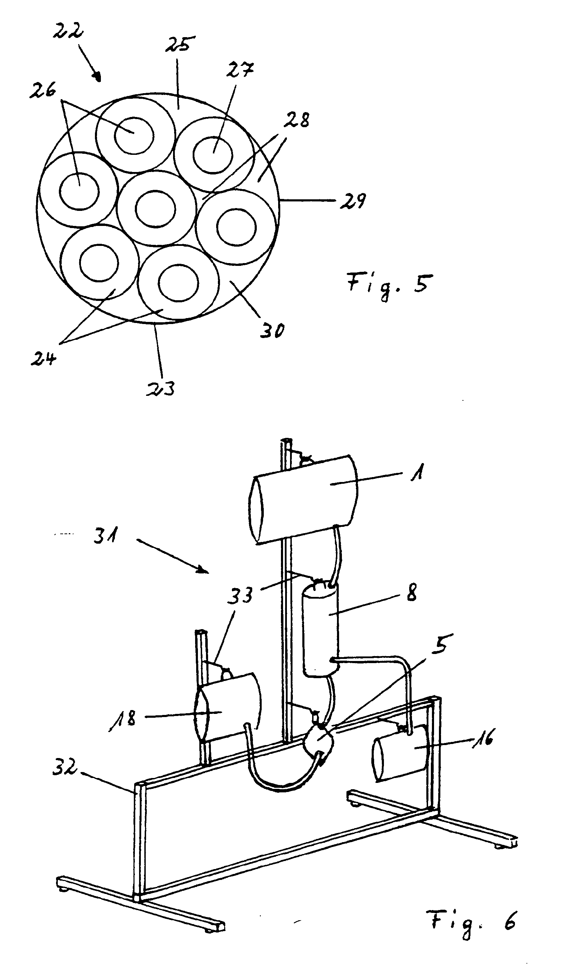

Die Fig. 5 zeigt eine Aufsicht auf den Querschnitt durch einen Hohlfaserfilter 22 in schematischer Darstellung, der aus einer Vielzahl von in einem festen Gehäuse 23 in Flußrichtung des Blutes angeordneten Hohlfasern 24 besteht, die an ihrem in Flußrichtung des Blutes unteren Ende mit ihren Außenwänden flüssigkeitsdicht in den Öffnungen einer Lochplatte 25 verklebt sind, so daß ihre Innenbereiche 26 insgesamt eine sich bis in den unteren Teil des Gehäuses 23 erstreckende Einlaßkammer 27 und die Gesamtheit ihrer Außenbereiche 28 eine an die Außenwand 29 des Gehäuses 23 reichende Auslaßkammer 30 ausbilden, an die in bekannter, luft- und keimdichter Weise Leitungen 13, 15, 17 anschließbar sind.

Die Fig. 6 zeigt die schematische isometrische Darstellung einer unveränderlichen Haltevorrichtung 31 für vorgegebene Positionen einer Vorrichtung zum Auftrennen von Blut in einzelne und/oder Gruppen seiner Bestandteile, die im vorliegenden Fall aus einem starren Gestell 32 mit relativ zu diesem lokal fixierten Halteelementen 33 in Form von Haken vorgegebener Form und Länge besteht, so daß die an diesen in vorgegebener Anordnung, die beispielsweise durch am Gestell angebrachte Symbole charakterisierbar ist, anbringbaren Elemente der in Rede stehenden Vorrichtung, beispielsweise Sammelbehälter 1, Filteranordnung 8, Aufnahmebehälter für Blutplasma 16, Leukozytendepletionsfilter 5 und Aufnahmebehälter für zelluläre Bestandteile des Blutes 18, sich relativ zum Gestell 32 stets an gleichem Ort und in vorgegebenen senkrechten Abständen zueinander befinden und so auch stets einen einmal vorgegebenen Rückstau in der Filteranordnung 8 erzeugen. Damit ist dann eine eindeutige Funktionsweise der gesamten vorliegenden Vorrichtung sichergestellt. Die Haltevorrichtung 31 bzw. das Gestell 32 kann dabei selbstverständlich jede geignete und dem Fachmann geläufige Form aufweisen und darüber hinaus auch transportierbar und zu diesem Zweck zusammenlegbar sein, solange sie im Betriebsfall unveränderlich bleibt.

Das vorliegende Patentbegehren bezieht sich selbstverständlich nicht allein auf die explizit dargestellten und beschriebenen Ausführungsbeispiele, sondern auf alle denkbaren Ausführungsformen einer erfindungsgemäßen Vorrichtung, die von dem Schutzumfang des Patentanspruchs 1 der vorliegenden Patentanmeldung erfaßt werden.

### Bezugszeichenliste

- 1: Sammelbehälter
- 2: Zuleitung
- 3: Ableitung
- 4: Verbindungselement/Kanüle
- 5: Leukozytendepletionsfilter
- 6: Verlängerung der Ableitung
- 7: Transferbehälter
- 8: Filteranordnung
- 9: Rückschlagventil
- 10: Bypass-Leitung
- 11,13: Verbindungsleitungen
- 12: sterile Kupplungsvorrichtung
- 14: Stabilisatorlösung
- 15: Ablaufleitung der Auslaßkammer
- 16: Aufnahmebehälter für Blutplasma
- 17: Ablaufleitung der Einlaßkammer
- 18: Aufnahmebehälter für zelluläre Bestandteile des Blutes
- 19: einstellbare Klemmvorrichtung/Rollklemme
- 20: Einrichtung zur Erzeugung eines Rückstaus
- 21: Additivlösung
- 22: Hohlfaserfilter
- 23: Gehäuse
- 24: Hohlfasern
- 25: Lochplatte
- 26: Innenbereiche
- 27: Einlaßkammer
- 28: Außenbereiche
- 29: Außenwand
- 30: Auslaßkammer
- 31: unveränderliche Haltevorrichtung
- 32: Gestell
- 33: Halteelemente (Haken)

## Patentansprüche

1. Vorrichtung zum Auftrennen von Blut in einzelne und/oder Gruppen seiner Bestandteile in Form eines sterilisierten geschlossenen Systems bestehend aus einem mit einer Zuleitung und einer Ableitung versehenen Sammelbehälter (1), dessen Zuleitung an ihrem dem Sammelbehälter abgewandten Ende mit einem mit einem oder mehreren Blutgefäßen eines Spenders koppelbaren Verbindungselement versehen ist, einer an dem dem Sammelbehälter abgewandten Ende der Ableitung mit dieser verbundenen Filteranordnung (8) mit einer Einlaß-(27) und einer Außlasskammer (28), an die jeweils eine Ablaufleitung angeschlossen ist, einem mit dem der Filteranordnung abgekehrten Ende der Ablaufleitung der Außlasskammer verbundenen Aufnahmebehälter (16) für Blutplasma sowie einem mit dem der Filteranordnung abgekehrten Ende der Ablaufleitung der Einlaßkammer verbundenen Aufnahmebehälter (18) für zelluläre Bestandteile des Blutes, wobei im Leitungsweg (3, 6, 11, 13, 15) zwischen Sammelbehälter (1) und Aufnahmebehälter für zelluläre Bestandteile des Blutes (18) ein Leukozytendepletionsfilter (5) ebenfalls bekannter Art angeordnet ist und dass der Leitungsweg (17) zwischen Filteranordnung (8) und Aufnahmebehälter für zelluläre Bestandteile des Blutes (18) eine Einrichtung zur Erzeugung eines Rückstaus (20) in der Filteranordnung (8) aufweist,
**dadurch gekennzeichnet,**
**dass** die Filteranordnung (8) ein mit einer blutverträglichen Flüssigkeit durchtränkter Hohlfaserfilter (22) bekannter Art ist, wobei die Gesamtheit der Innenbereiche (26) der Hohlfasern (24) die Einlaßkammer (27) und die Gesamtheit der Außenbereiche (28) der Hohlfasern (24) die Auslaßkammer (30) ausbildet.

2. Vorrichtung nach Anspruch 1,
**dadurch gekennzeichnet,**
**dass** das mit einem oder mehreren Blutgefäßen eines Spenders koppelbare Verbindungselement (4) eine Kanäle herkömmlicher Art ist.

3. Vorrichtung nach Anspruch 1 oder 2,
**dadurch gekennzeichnet,**
**dass** der Sammelbehälter (1) sowie die Aufnahmebehälter für Blutplasma und zelluläre Bestandteile des Blutes (16, 18) beutelartige Hohlkörper aus elastischer Kunststoff-Folie bekannter Art und die Zu-, Ab- und Ablaufleitungen (2, 3, 6, 11, 13, 15, 17) schlauchartige Verbindungselemente aus ebenfalls elastischem Kunststoffmaterial bekannter Art sind, wobei die Behälter (1, 7, 16, 18) und die Leitungen (2, 3, 6, 11, 13, 15, 17) miteinander luft- und keimdicht verschweißt oder mittels aufbrechbarer Ports und Steckverbindungen ebenfalls bekannter Art keimfrei und luftdicht verbindbar sind.

4. Vorrichtung nach einem der Ansprüche 1 bis 3,
**dadurch gekennzeichnet,**
**dass** die Filteranordnung (8) und der Leukozytendepletionsfilter (5) luftdicht und keimfrei mit ihren zu- und abführenden Leitungen (3, 6, 13, 15, 17) verbunden sind.

5. Vorrichtung nach einem der Ansprüche 1 bis 4,
**dadurch gekennzeichnet,**
**dass** die blutverträgliche Flüssigkeit physiologische Kochsalzlösung ist.

6. Vorrichtung nach einem der Anspruche 1 bis 4,
**dadurch gekennzeichnet,**
**dass** die blutverträgliche Flüssigkeit eine Stabilisatorlösung (14) bekannter Art ist.

7. Vorrichtung nach einem der Ansprüche 1 bis 6,
**dadurch gekennzeichnet,**
**dass** der sammelbehälter (1) mindestens im Startzeitpunkt der Blutaufnahme eine vorgegebene Menge einer Stabilisatorflüssigkeit (14) bekannter Art enthält.

8. Vorrichtung nach Anspruch 6 und 7,
**dadurch gekennzeichnet,**
**dass** die Stabilisatorlösung (14) mit der der Hohlfaserfilter (22) durchtränkt ist, dieselbe ist, die der Sammelbehälter (1) enthält.

9. Vorrichtung nach einem der Ansprüche 1 bis 8,
**dadurch gekennzeichnet,**
**dass** der Leukozytendepletionsfilter (5) in der Ableitung (3) des Sammelbehälters (1) zur Filteranordnung (8) angeordnet ist.

10. Vorrichtung nach Anspruch 9,
**dadurch gekennzeichnet,**
**dass** als Einrichtung zur Erzeugung eines Rückstaus (20) in der Filteranordnung (8) an der Ablaufleitung (17) von deren Einlaßkammer (27) zum Aufnahmebehälter für zelluläre Bestandteile des Blutes (18) eine einstellbare Klemmvorrichtung (19) angeordnet ist.

11. Vorrichtung nach Anspruch 10,
**dadurch gekennzeichnet,**
**dass** die einstellbare Klemmvorrichtung (19) eine Rollklemme bekannter Art ist.

12. Vorrichtung nach einem der Ansprüche 9 bis 11,
**dadurch gekennzeichnet,**
**dass** in der Verbindungsleitung (6, 11, 13) zwischen Leukozytendepletionsfilter (5) und Filteranordnung (8) ein zusätzlicher Transferbehälter (7) angeordnet ist.

13. Vorrichtung nach Anspruch 12,
**dadurch gekennzeichnet,**
**dass** der Transferbehälter (7) ein beutelartiger Hohlkörper aus elastischer Kunststoff-Folie bekannter Art ist.

14. Vorrichtung nach Anspruch 12 oder 3,
**dadurch gekennzeichnet,**
**dass** die Verbindungsleitung (11, 13) zwischen Transferbehälter (7) und Filteranordnung (8) eine Unterbrechung aufweist, die mittels einer sterilen Kupplungsvorrichtung (12) bekannter Art überbrückbar ist.

15. Vorrichtung nach einem der Ansprüche 1 bis 8,
**dadurch gekennzeichnet,**
**dass** der Leukozytendepletionsfilter (5) in der Ablaufleitung (17) der Einlaßkammer (27) der Filteranordnung (8) zum Aufnahmebehälter für zelluläre Bestandteile des Blutes (18) angeordnet ist.

16. Vorrichtung nach Anspruch 15,
**dadurch gekennzeichnet,**
**dass** der Leukozytendepletionsfilter (5) gleichzeitig die Einrichtung zur Erzeugung eines Rückstaus (20) in der Filteranordnung (8) ausbildet.

17. Vorrichtung nach Anspruch 15,
**dadurch gekennzeichnet,**
**dass** der Leukozytendepletionsfilter (5) in Verbindung mit einer vorgegebenen Anordnung der senkrechten Abstände zwischen Sammelbehälter (1), Filteranordnung (8), Leukozytendepletionsfilter (5), Aufnahmebehälter für Blutplasma (16) und Aufnahmebehälter für zelluläre Bestandteile des Blutes (18) die Einrichtung zur Erzeugung eines Rückstaus (20) in der Filteranordnung (8) ausbildet.

18. Vorrichtung nach Anspruch 17,
**dadurch gekennzeichnet,**
**dass** die vorgegebene Anordnung der senkrechten Abstände mittels einer wiederverwendbaren und zumindest während der Blutaufnahme unveränderlichen Haltevorrichtung (31) mit definiert angeordneten Halteelementen (33) für die Behälter (1, 7, 16, 18) und Filtereinrichtungen (5, 8) festlegbar ist.

19. Vorrichtung nach Anspruch 18,
**dadurch gekennzeichnet,**
**dass** die Haltevorrichtung (31) transportabel ist.

20. Vorrichtung nach Anspruch 18 oder 19,
**dadurch gekennzeichnet,**
**dass** die Haltevorrichtung (31) für Transportzwecke zusammenlegbar ist.

21. Vorrichtung nach einem der Ansprüche 18 bis 20,
**dadurch gekennzeichnet,**
**dass** die Halteelemente (33) Konnzeichnungen für die eindeutige Zuordnung der einzelnen Behälter (1, 7, 16, 18) und Filtereinrichtungen (5, 6) aufweisen.

22. Vorrichtung nach einem der Ansprüche 18 bis 21,
**dadurch gekennzeichnet,**
**dass** die Halteelemente (33) eine haken- und/oder gabelartige Gestalt aufweisen.

23. Vorrichtung nach einem der Ansprüche 18 bis 22,
**dadurch gekennzeichnet,**
**dass** die Haltevorrichtung (31) eine rahmenartige Ausbildung aufweist.

24. Vorrichtung nach einem der Ansprüche 18 bis 22,
**dadurch gekennzeichnet,**
**dass** die Haltevorrichtung (31) eine baumartige Ausbildung aufweist.

25. Vorrichtung nach einem der Ansprüche 15 bis 24,
**dadurch gekennzeichnet,**
**dass** in der Verbindungsleitung (17) zwischen Filteranordnung (8) und Leukozytendepletionsfilter (5) ein zusätzlicher Transferbehälter (7) angeordnet ist.

26. Vorrichtung nach Anspruch 25,
**dadurch gekennzeichnet,**
**dass** der Transferbehälter (7) während der Blutaufnahme mit vorgegebenen senkrechten Abständen zur Filteranordnung (8) einerseits und zum Leukozytendepletionsfilter (5) andererseits angeordnet ist.

27. Vorrichtung nach Anspruch 25 oder 26,
**dadurch gekennzeichnet,**
**dass** der Transferbehälter (7) ein beutelartiger Hohlkörper aus elastischer Kunststoff-Folie bekannter Art ist.

28. Vorrichtung nach einem der Ansprüche 25 bis 27,
**dadurch gekennzeichnet,**
**dass** die Verbindungsleitung (17) zwischen Transferbehälter (7) und Leukozytendepletionsfilter (5) eine Unterbrechung aufweist, die mittels einer sterilen Kupplungsvorrichtung (12) bekannter Art überbrückbar ist.

29. Vorrichtung nach einem der Ansprüche 15 bis 28,
**dadurch gekennzeichnet,**
**dass** am Leitungsweg (17) von der Filteranordnung (8) zum Aufnahmebehälter für zelluläre Bestandteile des Blutes (18) eine einstellbare Klemmvorrichtung (19) als weitere Einrichtung zur Erzeugung eines Rückstaus (20) in der Filteranordnung (8) angeordnet ist.

30. Vorrichtung nach Anspruch 29,
**dadurch gekennzeichnet,**
**dass** die einstellbare Klemmvorrichtung (19) eine Rollklemme bekannter Art ist.

31. Vorrichtung nach einem der Ansprüche 1 bis 30,
**dadurch gekennzeichnet,**
**dass** parallel zum Leukozytendepletionsfilter (5) eine Bypass-Leitung (10) mit einem Rückschlagventil (9) angeordnet ist, wobei das Rückschlagventil (9) entgegen der für die Aufnahme und Auftrennung des Blutes vorgesehenen Fließrichtung durch den Leukozytendepletionsfilter (5) öffnet.

32. Vorrichtung nach einem der Ansprüche 1 bis 31,
**dadurch gekennzeichnet,**
**dass** parallel zur Filteranordnung (8) eine Bypass-Leitung (10) mit einem Rückschlagventil (9) angeordnet ist, wobei das Rückschlagventil (9) entgegen der für die Aufnahme und Auftrennung des Blutes vorgesehenen Fließrichtung durch die Filteranordnung (8) öffnet.

33. Vorrichtung nach einem der Ansprüche 1 bis 32,
**dadurch gekennzeichnet,**
**dass** der Aufnahmebehälter für zelluläre Bestandteile des Blutes (18) mit einem luft- und keimdicht abtrennbaren Entlüftungsbehälter versehen ist.

34. Vorrichtung nach einem der Ansprüche 1 bis 33,
**dadurch gekennzeichnet,**
**dass** der Aufnahmebehälter für zelluläre Bestandteile des Blutes (18) eine vorfüllung mit einer vorgegebenen Menge einer Additivlösung (21) bekannter Art aufweist.

## Claims

1. Device for separating blood into individual components and/or groups of its components, in the form of a sterilized, closed system comprising: a collecting container (1) which is provided with a delivery line and a discharge line and whose delivery line is connected, at its end remote from the collecting container, to a connection element which can be coupled to one or more blood vessels of a donor; a filter assembly (8) which is connected to the discharge line, at the end thereof remote from the collecting container, said filter assembly (8) having an inlet chamber (27) and an outlet chamber (28) to each of which a respective drain line is attached; a receptacle (16) for blood plasma connected to that end of the discharge line of the outlet chamber directed away from the filter assembly, and a receptacle (18) for cellular components of the blood which is connected to that end of the discharge line of the inlet chamber directed away from the filter assembly, a leukocyte depletion filter (5) likewise of a known type being arranged in the conduit path (3, 6, 11, 13, 15) between the collecting container (1) and the receptacle (18) for cellular components of the blood, and the conduit path (17) between the filter assembly (8) and the receptacle (18) for cellular components of the blood having a means for generating a backflow (20) in the filter assembly (8), **characterized in that** the filter assembly (8) is a hollow-fibre filter (22) of a known type saturated with a blood-compatible liquid, the totality of the inner areas (26) of the hollow fibres (24) forming the inlet chamber (27) and the totality of the outer areas (28) of the hollow fibres (24) forming the outlet chamber (30).

2. Device according to Claim 1, **characterized in that** the connection element (4) which can be coupled to one or more blood vessels of a donor is a cannula of a conventional type.

3. Device according to Claim 1 or 2, **characterized in that** the collecting container (1) and the receptacles (16, 18) for blood plasma and cellular components of the blood are bag-like hollow bodies made of elastic plastic film of a known type, and the delivery, discharge and drain lines (2, 3, 6, 11, 13, 15, 17) are tubular connection elements made of similarly elastic plastic material of a known type, the containers (1, 7, 16, 18) and the lines (2, 3, 6, 11, 13, 15, 17) being welded to one another in an airtight and germ-proof manner or being able to be connected to one another in a germ-free and airtight manner by means of openable ports and plug connections.

4. Device according to one of Claims 1 to 3, **characterized in that** the filter assembly (8) and the leukocyte depletion filter (5) are connected in an airtight and germ-free manner to their delivery and discharge lines (3, 6, 13, 15, 17).

5. Device according to one of Claims 1 to 4, **characterized in that** the blood-compatible liquid is physiological saline solution.

6. Device according to one of Claims 1 to 4, **characterized in that** the blood-compatible liquid is a stabilizer solution (14) of a known type.

7. Device according to one of Claims 1 to 6, **characterized in that** the collecting container (1) contains a predetermined amount of a stabilizer liquid (14) of a known type, at least at the time when the collecting of blood starts.

8. Device according to Claims 6 and 7, **characterized in that** the stabilizer solution (14) with which the hollow-fibre filter (22) is saturated is the same one that is contained in the collecting container (1).

9. Device according to one of Claims 1 to 8, **characterized in that** the leukocyte depletion filter (5) is arranged in the discharge line (3) from the collecting container (1) to the filter assembly (8).

10. Device according to Claim 9, **characterized in that**, as the means for generating a backflow (20) in the filter arrangement (8), an adjustable clamping device (19) is arranged on the discharge line (17) from the inlet chamber (27) to the receptacle (18) for cellular components of the blood.

11. Device according to Claim 10, **characterized in that** the adjustable clamping device (19) is a roller clamp of a known type.

12. Device according to one of Claims 9 to 11, **characterized in that** an additional transfer container (7) is arranged in the connection line (6, 11, 13) between leukocyte depletion filter (5) and filter arrangement (8).

13. Device according to Claim 12, **characterized in that** the transfer container (7) is a bag-like hollow body made from elastic plastic film of a known type.

14. Device according to Claim 12 or 13, **characterized in that** the connection line (11, 13) between transfer container (7) and filter assembly (8) has a gap which can be bridged by means of a sterile coupling device (12) of a known type.

15. Device according to one of Claims 1 to 8, **characterized in that** the leukocyte depletion filter (5) is arranged in the drain line (17) from the inlet chamber (27) of the filter assembly (8) to the receptacle (18) for cellular components of the blood.

16. Device according to Claim 15, **characterized in that** the leukocyte depletion filter (5) at the same time forms the means for generating a backflow (20) in the filter assembly (8).

17. Device according to Claim 15, **characterized in that** the leukocyte depletion filter (5), in conjunction with a predetermined arrangement of the vertical distances between collecting container (1), filter assembly (8), leukocyte depletion filter (5), receptacle (16) for blood plasma and receptacle (18) for cellular components of the blood, forms the means for generating a backflow (20) in the filter assembly (8).

18. Device according to Claim 17, **characterized in that** the predetermined arrangement of the vertical distances can be fixed by means of a reusable holder apparatus (31) which is unalterable at least during the blood collection and which has holding elements (33) arranged in a defined way for the containers (1, 7, 16, 18) and filter means (5, 8).

19. Device according to Claim 18, **characterized in that** the holding apparatus (31) is transportable.

20. Device according to Claim 18 or 19, **characterized in that** the holding apparatus (31) can be folded up for transport purposes.

21. Device according to one of Claims 18 to 20, **characterized in that** the holding elements (33) have markings for unambiguous allocation of the individual containers (1, 7, 16, 18) and filter means (5, 6).

22. Device according to one of Claims 18 to 21, **characterized in that** the holding elements (33) have a hook-like and/or fork-like configuration.

23. Device according to one of Claims 18 to 22, **characterized in that** the holding apparatus (31) has a frame-like configuration.

24. Device according to one of Claims 18 to 22, **characterized in that** the holding apparatus (31) has a tree-like configuration.

25. Device according to one of Claims 15 to 24, **characterized in that** an additional transfer container (7) is arranged in the connection line (17) between filter assembly (8) and leukocyte depletion filter (5).

26. Device according to Claim 25, **characterized in that**, during collection of blood, the transfer container (7) is arranged at predetermined vertical distances from the filter assembly (8) on the one hand and the leukocyte depletion filter (5) on the other hand.

27. Device according to Claim 25 or 26, **characterized in that** the transfer container (7) is a bag-like hollow body made from elastic plastic film of a known type.

28. Device according to one of Claims 25 to 27, **characterized in that** the connection line (17) between transfer container (7) and leukocyte depletion filter (5) has a gap which can be bridged by means of a sterile coupling device (12) of a known type.

29. Device according to one of Claims 15 to 28, **characterized in that**, on the conduit path (17) from the filter assembly (8) to the receptacle (18) for cellular components of the blood, an adjustable clamping device (19) is arranged as a further means for generating a backflow (20) in the filter assembly (8).

30. Device according to Claim 29, **characterized in that** the adjustable clamping device (19) is a roller clamp of a known type.

31. Device according to one of Claims 1 to 30, **characterized in that** a bypass line (10) with a nonreturn valve (9) is arranged parallel to the leukocyte depletion filter (5), and said nonreturn valve (9) opens counter to the direction of flow provided for reception and separation of the blood via the leukocyte depletion filter (5).

32. Device according to one of Claims 1 to 31, **characterized in that** a bypass line (10) with a nonreturn valve (9) is arranged parallel to the filter assembly (8), and said nonreturn valve (9) opens counter to the direction of flow provided for reception and separation of the blood via the filter assembly (8).

33. Device according to one of Claims 1 to 32, **characterized in that** the receptacle (18) for cellular components of the blood is provided with an airtight and germ-proof detachable venting container.

34. Device according to one of Claims 1 to 33, **characterized in that** the receptacle (18) for cellular components of the blood is prefilled with a predetermined amount of an additive solution (21) of a known type.

## Revendications

1. Dispositif pour séparer le sang en ses composants individuels et/ou en groupes de ses composants sous forme d'un système fermé stérilisé constitué d'un récipient collecteur (1) pourvu d'une conduite d'amenée et d'une conduite de sortie dont la conduite d'amenée est pourvue à son extrémité opposée au récipient collecteur d'un élément de connexion pouvant être accouplé à un ou plusieurs vaisseaux sanguins d'un donneur, d'un agencement de filtre (8) à l'extrémité de la conduite de sortie opposée au récipient collecteur, connecté à celle-ci, avec une chambre d'entrée (27) et une chambre de sortie (28), à laquelle est raccordée une conduite d'écoulement respective, d'un récipient de réception (16) connecté à l'extrémité de la conduite d'écoulement de la chambre de sortie opposée à l'agencement de filtre, pour le plasma sanguin ainsi que d'un récipient de réception (18) connecté à l'extrémité de la conduite d'écoulement de la chambre d'entrée opposée à l'agencement de filtre, pour les composants cellulaires du sang, un filtre destiné à réaliser la déplétion des leucocytes (5), également de type connu, étant disposé dans le parcours de la conduite (3, 6, 11, 13, 15) entre le récipient collecteur (1) et le récipient de réception pour les composants cellulaires du sang (18), et le parcours de la conduite (17) entre l'agencement de filtre (8) et le récipient de réception pour les composants cellulaires du sang (18) présentant un dispositif pour produire une obstruction (20) dans l'agencement de filtre (8),
**caractérisé en ce que**
l'agencement de filtre (8) est un filtre à fibres creuses (22) imbibé de liquide compatible avec le sang de type connu, la totalité des régions internes (26) des fibres creuses (24) constituant la chambre d'entrée (27) et la totalité des régions externes (28) des fibres creuses (24) constituant la chambre de sortie (30).

2. Dispositif selon la revendication 1,
**caractérisé en ce que**
l'élément de connexion (4) pouvant être accouplé à un ou plusieurs vaisseaux sanguins d'un donneur est une canule de type usuel.

3. Dispositif selon la revendication 1 ou 2,
**caractérisé en ce que**
le récipient collecteur (1) ainsi que les récipients de réception pour le plasma sanguin et les composants cellulaires du sang (16, 18) sont des corps creux de type sachet en feuille de plastique élastique de type connu et les conduites d'amenée, de sortie et d'écoulement (2, 3, 6, 11, 13, 15, 17) sont des éléments de connexion de type tuyau flexible également en matériau plastique élastique de type connu, les récipients (1, 7, 16, 18) et les conduites (2, 3, 6, 11, 13, 15, 17) étant soudés les uns aux autres de manière étanche à l'air et aux germes ou pouvant être connectés de manière étanche à l'air et exempte de germes au moyen de connexions enfichables et d'orifices perçables également de type connu.

4. Dispositif selon l'une quelconque des revendications 1 à 3,
**caractérisé en ce que**
l'agencement de filtre (8) et le filtre destiné à réaliser la déplétion des leucocytes (5) sont connectés de manière étanche à l'air et exempte de germes à leurs conduites d'amenée et de sortie (3, 6, 13, 15, 17).

5. Dispositif selon l'une quelconque des revendications 1 à 4,
**caractérisé en ce que**
le liquide compatible avec le sang est une solution saline physiologique.

6. Dispositif selon l'une quelconque des revendications 1 à 4,
**caractérisé en ce que**
le liquide compatible avec le sang est une solution stabilisatrice (14) de type connu.

7. Dispositif selon l'une quelconque des revendications 1 à 6,
**caractérisé en ce que**
le récipient collecteur (1) contient au moins au point de départ de la collecte de sang, une quantité prédéfinie d'un liquide stabilisateur (14) de type connu.

8. Dispositif selon les revendications 6 et 7,
**caractérisé en ce que**
la solution stabilisatrice (14) avec laquelle le filtre à fibres creuses (22) est imbibé est celle que contient le récipient collecteur (1).

9. Dispositif selon l'une quelconque des revendications 1 à 8,
**caractérisé en ce que**
le filtre destiné à réaliser la déplétion des leucocytes (5) est disposé dans la conduite de sortie (3) du récipient collecteur (1) vers l'agencement de filtre (8).

10. Dispositif selon la revendication 9,
**caractérisé en ce qu'**
un dispositif de serrage (19) ajustable est disposé en tant que dispositif pour produire une obstruction (20) dans l'agencement de filtre (8) sur la conduite d'écoulement (17) avant sa chambre d'entrée (27) vers le récipient de réception pour les composants cellulaires du sang (18).

11. Dispositif selon la revendication 10,
**caractérisé en ce que**
le dispositif de serrage ajustable (19) est une pince à rouleaux de type connu.

12. Dispositif selon l'une quelconque des revendications 9 à 11,
**caractérisé en ce qu'**
un récipient de transfert (7) supplémentaire est disposé dans la conduite de connexion (6, 11, 13) entre le filtre destiné à réaliser la déplétion des leucocytes (5) et l'agencement de filtre (8).

13. Dispositif selon la revendication 12,
**caractérisé en ce que**
le récipient de transfert (7) est un corps creux de type sachets en feuille de plastique élastique de type connu.

14. Dispositif selon la revendication 12 ou 13,
**caractérisé en ce que**
la conduite de connexion (11, 13) entre le récipient de transfert (7) et l'agencement de filtre (8) présente une interruption qui peut être pontée par un dispositif d'accouplement stérile (12) de type connu.

15. Dispositif selon l'une quelconque des revendications 1 à 8,
**caractérisé en ce que**
le filtre destiné à réaliser la déplétion des leucocytes (5) est disposé dans la conduite d'écoulement (17) de la chambre d'entrée (27) de l'agencement de filtre (8) vers le récipient de réception pour les composants cellulaires du sang (18).

16. Dispositif selon la revendication 15,
**caractérisé en ce que**
le filtre destiné à réaliser la déplétion des leucocytes (5) constitue en même temps le dispositif pour produire une obstruction (20) dans l'agencement de filtre (8).

17. Dispositif selon la revendication 15,
**caractérisé en ce que**
le filtre destiné à réaliser la déplétion des leucocytes (5), conjointement avec un agencement prédéfini des espacements verticaux entre le récipient de collecte (1), l'agencement de filtre (8), le filtre destiné à réaliser la déplétion des leucocytes (5), le récipient de réception pour le plasma sanguin (16) et le récipient de réception pour les composants cellulaires du sang (18) constitue le dispositif pour produire une obstruction (20) dans l'agencement de filtre (8).

18. Dispositif selon la revendication 17,
**caractérisé en ce que**
l'agencement prédéfini des espacements verticaux peut être établi au moyen d'un dispositif de fixation (31) réutilisable et immuable au moins pendant la collecte du sang avec des éléments de fixation (33) disposés de manière définie pour les récipients (1, 7, 16, 18) et les dispositifs de filtration (5, 8).

19. Dispositif selon la revendication 18,
**caractérisé en ce que**
le dispositif de fixation (31) est transportable.

20. Dispositif selon la revendication 18 ou 19,
**caractérisé en ce que**
le dispositif de fixation (31) peut être replié pour son transport.

21. Dispositif selon l'une quelconque des revendications 18 à 20,
**caractérisé en ce que**
les éléments de fixation (33) présentent des indications pour l'affectation univoque des récipients individuels (1, 7, 16, 18) et des dispositifs de filtration (5, 6).

22. Dispositif selon l'une quelconque des revendications 18 à 21,
**caractérisé en ce que**
les éléments de fixation (33) présentent une forme de type crochet et/ou fourche.

23. Dispositif selon l'une quelconque des revendications 18 à 22,
**caractérisé en ce que**
le dispositif de fixation (31) présente une réalisation de type cadre.

24. Dispositif selon l'une quelconque des revendications 18 à 22,
**caractérisé en ce que**
le dispositif de fixation (31) présente une réalisation de type arbre.

25. Dispositif selon l'une quelconque des revendications 15 à 24,
**caractérisé en ce qu'**
un dispositif de transfert (7) supplémentaire est disposé dans la conduite de connexion (17) entre l'agencement de filtre (8) et le filtre destiné à réaliser la déplétion des leucocytes (5).

26. Dispositif selon la revendication 25,
**caractérisé en ce que**
le récipient de transfert (7) est disposé pendant la collecte du sang avec des espacements verticaux prédéfinis par rapport à l'agencement de filtre (8) d'une part et par rapport au filtre destiné à réaliser la déplétion des leucocytes (5) d'autre part.

27. Dispositif selon la revendication 25 ou 26,
**caractérisé en ce que**
le récipient de transfert (7) est un corps creux de type sachet en feuille de plastique élastique de type connu.

28. Dispositif selon l'une quelconque des revendications 25 à 27,
**caractérisé en ce que**
la conduite de connexion (17) entre le récipient de transfert (7) et le filtre destiné à réaliser la déplétion des leucocytes (5) présente une interruption qui peut être pontée par un dispositif d'accouplement stérile (12) de type connu.

29. Dispositif selon l'une quelconque des revendications 15 à 28,
**caractérisé en ce que**
sur le parcours de la conduite (17) de l'agencement de filtre (8) vers le récipient de réception pour les composants cellulaires du sang (18), est disposé un dispositif de serrage ajustable (19) en tant que dispositif supplémentaire pour produire une obstruction (20) dans l'agencement de filtre (8).

30. Dispositif selon la revendication 29,
**caractérisé en ce que**
le dispositif de serrage ajustable (19) est une pince à rouleaux de type connu.

31. Dispositif selon l'une quelconque des revendications 1 à 30,
**caractérisé en ce qu'**
une conduite de dérivation (10) avec un clapet anti-retour (9) est disposée parallèlement au filtre destiné à réaliser la déplétion des leucocytes (5), le clapet anti-retour (9) s'ouvrant à l'encontre de la direction d'écoulement à travers le filtre destiné à réaliser la déplétion des leucocytes (5) prévue pour la collecte et la séparation du sang.

32. Dispositif selon l'une quelconque des revendications 1 à 31,
**caractérisé en ce qu'**
une conduite de dérivation (10) avec un clapet anti-retour (9) est disposée parallèlement à l'agencement de filtre (8), le clapet anti-retour (9) s'ouvrant à l'encontre de la direction d'écoulement à travers l'agencement de filtre (8) prévue pour la collecte et la séparation du sang.

33. Dispositif selon l'une quelconque des revendications 1 à 32,
**caractérisé en ce que**
le récipient de réception pour les composants cellulaires du sang (18) est pourvu d'un récipient de désaérage pouvant être séparé de manière étanche à l'air et aux germes.

34. Dispositif selon l'une quelconque des revendications 1 à 33,
**caractérisé en ce que**
le récipient de réception pour les composants cellulaires du sang (18) présente un préremplissage avec une quantité prédéfinie d'une solution d'additif (21) de type connu.
